# EUROPEAN PATENT SPECIFICATION

(11) **EP 2 714 122 B1**
(45) Date of publication and mention of the grant of the patent: **08.07.2015**
(21) Application number: 12778401.5
(22) Date of filing: 24.05.2012
(51) Int. Cl.: A61M 1/00

(54) **NEGATIVE PRESSURE DEVICE FOR TREATING WOUNDS**
UNTERDRUCKVORRICHTUNG ZUR BEHANDLUNG VON WUNDEN
DISPOSITIF DE TRAITEMENT PAR PRESSION NÉGATIVE DESTINÉ À TRAITER LES PLAIES

(30) Priority: 24.05.2011 IT BO20110296
(43) Date of publication of application: 09.04.2014
(73) Proprietor: MET S.r.l., San Lazzaro Di Savena (IT)
(72) Inventor: MAZZONI, Paolo, I-47015 Modigliana (IT); BIANCHI, Tommaso, 40065 Pianoro (IT)
(74) Representative: Boggio, Luigi
(86) International application number: PCT/IB2012/052619
(87) International publication number: WO 2013/001382

(56) References cited:
- WO-A1-2006/105892
- WO-A1-2008/064502
- WO-A1-2009/049232
- WO-A2-2007/070697
- WO-A2-2009/114624
- GB-A- 2 423 019
- US-A1- 2007 161 937
- US-A1- 2007 219 497
- US-A1- 2008 275 409

## Description

### TECHNICAL FIELD

The present invention tackles the problem of treating wounds, in particular wide wounds or chronic wounds and pressure sores.

### BACKGROUND ART

The problem of treating this type of wounds is strongly felt, because population aging and the present epidemics of obesity and diabetes have increased the number of people affected. Moreover, the number of antibioticresistant microorganisms is increasing, and this kind of therapy is loosing efficacy. National health services have less and less resources, and therefore all the interventions facilitating and accelerating the procedures performed by health operators for treating wounds are welcome, especially systems allowing to perform wound treatment at patient's home instead of hospital treatment.

From WO 9309727 devices applying a pressure lower than chamber pressure to wounds are known. In this field, "reduced pressure", "vacuum treatment", "treatment under negative pressure" are synonyms.

In particular, WO 9309727 describes a device applying negative pressure to a wound. The device is inserted on the wound surface, and the wound sealed with a polymeric sheet adhering to the skin. Between the sheet and the wound surface a open-cell polymeric foam is inserted (screen means) in order to avoid wound hypertrophy. A problem linked to this solution is that tissues tend to grow within the polymeric foam itself, therefore when the dressing is changed, the patient feels a strong pain. The removal of the polymeric foam during dressing change can cause strong bleeding. Moreover, removing all the polymeric foam from the wound is not easy, and polymeric foam left in the wound can cause dangerous infections. These problems constitute the ground for a notification issued by FDA on November 13, 2009 (http://www.fda.gov/MedicalDevices/Safety/AlertsandNotices/ PublicHealthNotifications/ucm190658.htm), wherein 6 deaths and 77 injuries associated with Negative Pressure Wound Therapy systems are reported. Retention of foam dressing pieces and foam adhering to tissues or imbedded in the wound were noted in 32 injury reports.

Finally, said screen means are not easy to place in the wound: a certain manual ability is requested by the health operator cutting the polymeric material in a shape fit for the wound, and a considerable part of the time needed for dressing is spent in fitting such material to the wound shape.

In WO 2004/037334 and US 4382441 devices are described which, in addition to applying a negative pressure to the wound so as to drain liquids (exudates), can also irrigate the wound with liquids accelerating healing. In particular, in WO 2004/037334 inflatable hollow bodies are described, to be inserted between the wound and the wound sealing means.

In WO 2009/002260 a device for treating wounds with reduced pressure is described, comprising, as the already cited devices, a wound sealing film, a reduced pressure source, as well as a tube, characterized in that the interior of the tube comprises a longitudinal first strand made of a hydrophobic material, as well as a second strand, made of an open-pored hydrophilic material, extending longitudinally over at least a part of the length of the tube. Said material strands are enclosed in a tube casing made of a flexible material. The fluid-receiving element can be formed by rolling-up or folding of a long portion of the tube (Figure 14).

In US-A1-2008/0275409 a device for treating wounds with negative pressure is described.

Such device comprises:
- an adhesive film that defines a chamber in relation to the wound;
- a vacuum source which can be adjusted to produce negative pressures in the same chamber;
- a canister for collecting liquids drained from the wound;
- a reservoir containing medicated liquids to be instilled in the wound;
- an unit for setting both the negative pressure and the quantity of administered medication;
- a pressure sensor to detect the negative pressure present in the chamber;
- a diffuser mounted on the film, the diffuser being connected both to vacuum source and to a pump and comprising micro-valves for diffusing medicated liquids; and
- a pump administering medicated liquids exerting a pressure capable of opening the micro-valves.

### DISCLOSURE OF INVENTION, AS CLAIMED

Aim of the present invention is to provide a wound treatment device that can at the same time both establish a reduced pressure in the wound, and irrigate the wound with liquids and/or gases capable to accelerate its healing.

A second aim of the present invention consists in providing a device not needing screen means to be inserted between the wound and the tube applying vacuum in order to avoid wound hypertrophy.

A further aim of the present invention consists in providing a simplified device allowing to perform wound treating at home, facilitating as much as possible wound dressing to the health operator, decreasing the time necessary for dressing.

The whole device applied to the patient comprises:
- a vacuum source producing a negative pressure adjustable between 0 and at least -200 mm Hg, so that pressure increases very gradually, without causing pain to the patient;
- a pump administering medication of different nature (e.g. antibiotics, pain-relievers, anti-inflammatory, cellular proliferation stimulants, dermal matrix components, vascular growth factors, etc.); the pump must be able to exert a pressure at least superior to 1 bar;
- a spiral diffuser, at the same time on one side diffusing medications in an uniform way with dosages inferior even to 1 ml, and on the other side suctioning so that the wound is kept under negative pressure; said spiral diffuser is connected on one side to the vacuum source, and on the other to the pump administering medicaments;
- an adhesive polyurethane film to seal and isolate the wound.

The vacuum source is a membrane pump electronically controlled through a sensor detecting the value of negative pressure. The pump is connected to a canister collecting the fluids drained from the wound; in the tube an antibacterial filter is inserted. In the canister there is a gelling agent converting the fluids drained from the wound into a gel, having a more agreeable aspect for the patient and necessary for the norms concerning hospital waste management. In the canister a tube coming from the wound, and another tube going to the vacuum source are inserted. A micro-hole in the sealing system allows a continuous flow of liquids and gas in the suction tube, avoiding the formation of stasis in the tube loops and controlling the functioning of the vacuum system. Preferably, the value of the negative pressure is not steady, but varies between two values set by the operator (e.g., -110 mm Hg to -130 mm Hg).

The pump for administering medication can be, for instance, a peristaltic volumetric pump administering a liquid volume varying between 0,1 ml/min and 5 ml/min. It is electronically controlled and can administer the volumes set by the operator in a given time (e.g. 1 ml every 2 hours).

The spiral diffuser is made of a rolled up flexible tube made of polymeric material, that can be unrolled and cut in order to fit it to wound dimensions. The tube exhibits hollow villi; in each villus a valve is present, through which the medication is administered. It is important to note that the valve present in each villus is normally closed and opens only when a positive pressure is exerted by the pump for administering medications. The micro-valves are closed if the negative pressure caused by the vacuum source or chamber pressure is acting. In this way very small quantities of medication can be administered, with the certainty to wet wound surface. At the center of the spiral diffuser there is the end of the suction tube, and the micro-hole allowing a constant active pressure flow. A pH-sensor is moreover present.

The polymeric polyurethane film is adhesive and able to adhere to the healthy wound borders. This guarantees vacuum in the wound.

### BRIEF DESCRIPTION OF THE DRAWINGS

The present invention will now be described in detail with the help of the following figures, showing:
Figure 1 A and B a schematic view of the spiral diffuser inserted in the wound;
Figure 2 the components of the device;
Figure 3 a block diagram of the device;
Figure 4 A and B detailed views of the spiral diffuser;
Figure 5 an inferior view of diffuser 11;
Figure 6 a vertical section of diffuser 11.

### BEST MODE FOR CARRYING OUT THE INVENTION

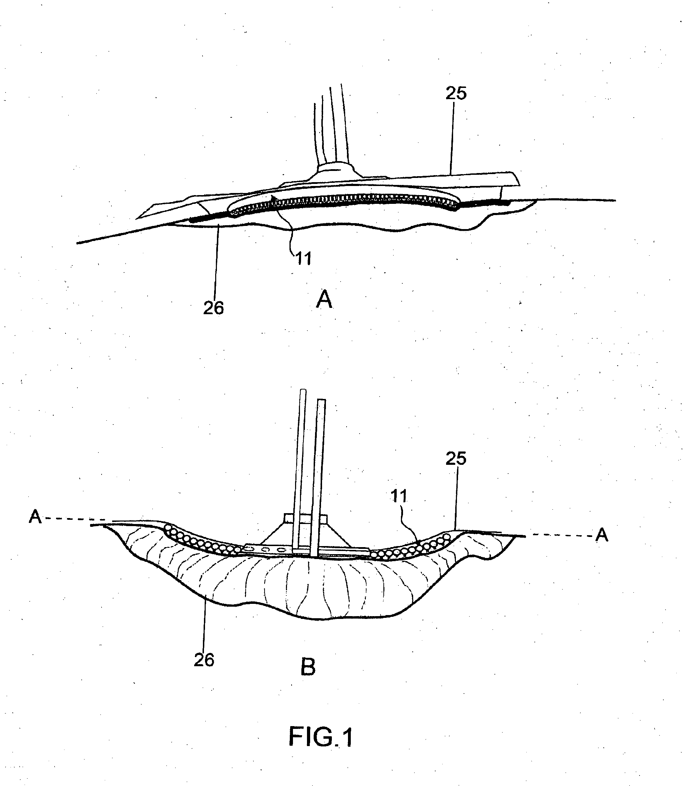

Figure 1 shows the positioning of spiral diffuser 11 in wound 26; spiral diffuser 11 is covered by polymeric polyurethane film 25 sealing wound 26.

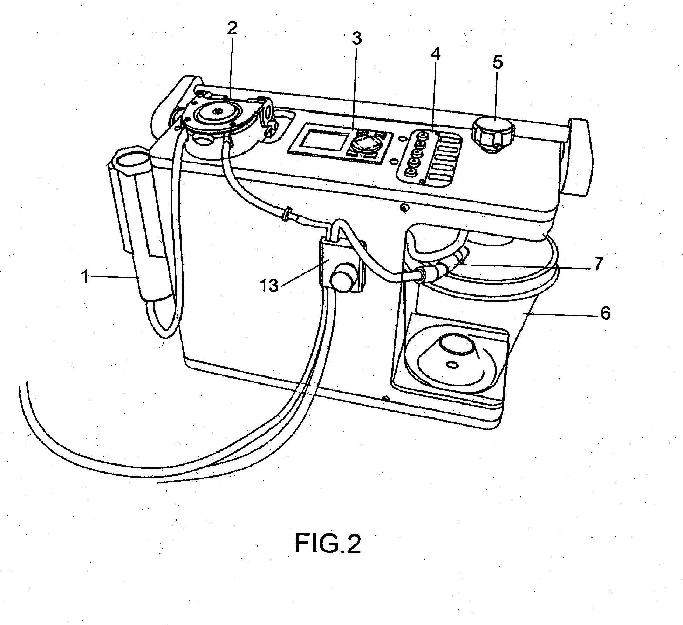

Figure 2 shows all the parts of the device: irrigating liquid reservoir 1, peristaltic pump 2, unit 3, with display, for programming the device, touches 4, knob 5 for blocking the drained liquid canister, canister 6 for liquids drained from wound 26, connection 7 to the vacuum pump, clamp 13 for single-use tubes.

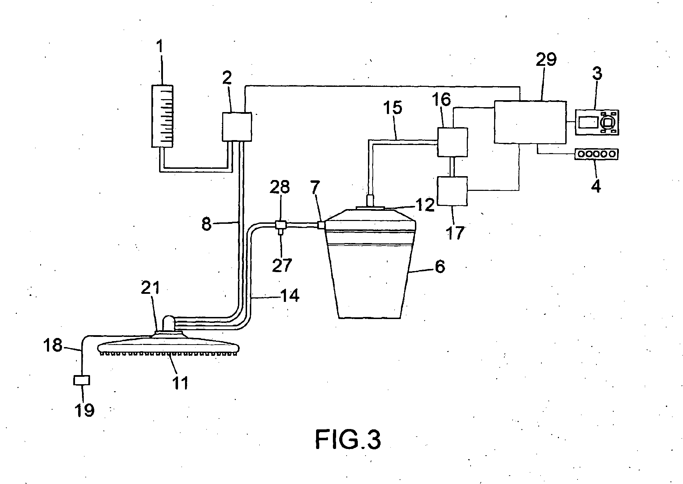

Figure 3 is a block diagram of the device, wherein 1 is the irrigating liquid reservoir, from which peristaltic pump 2 draws medicated liquid, sending it to spiral diffuser 11 through tube 8. The instilled medication can also be a liquid containing a gas.

The volume of instilled liquid and instillation times are programmed through unit 3. The wound (not shown in Figure 3) is kept under negative pressure through tube 14, which is connected to liquid-collecting canister 6 through connection 7. Indicatively, the drained liquid collecting canister can contain 800 ml. Negative pressure is generated through pump 16, connected to liquid-collecting canister 6 through tube 15 and antibacterial filter 12. The value of negative pressure is controlled through pressure sensor 17 and an electronic board 29. Once a given value of negative pressure is set, pressure sensor 17 will control the pump, through the electronic board 29, so that on canister 6, tube 7 and spiral diffuser 11 the desired level of negative pressure is obtained.

The quantity of medicated liquid administered must be very precise, and this is obtained through peristaltic pump 2 which is a volumetric pump, that at every turn of its axle can transfer a very small volume of liquid, in a very precise way. Pump 2 is controlled through programming panel 3, and at every turn can pump a minimum of 0,1 ml. The system can control the pump every half turn, so that the minimum administrable quantity is 0,05 ml. By controlling the number of turns and the speed of the pump, it is also possible to instill big quantities of liquid, for instance for cleansing the wound (e.g. 500 ml every 20 minutes). Suction manifold 21 is moreover connected to a capillary tube 18 at the end of which an antibacterial micro-filter 19 is present.

In this way a minimal quantity of atmospheric air can enter, so that a very small air flow is formed in tube 14, in order to avoid stasis formation in tube loops and to control the vacuum system, varying the value of negative pressure in a time controlled by the electronic system, which can detect an occlusion or a leakage in the system. On tube 14 an optical sensor 28 can also be inserted, detecting blood presence in the drained liquids. If blood in drained liquids is over a threshold level, this means that a bleeding is occurring. Vacuum pump then stops and the wound is automatically brought back to chamber pressure; moreover an acoustic/optic alarm is activated informing patient and health operator of the bleeding. Optionally also a sensor 27 detecting the status of the wound can be present, showing it on the unit display 3. The wound status can be communicated to the physician by a home-treated patient, too. Sensor 27 can be e.g. a pH-meter, analyzing liquids drained from the wound. In this case, too, alarms can be activated.

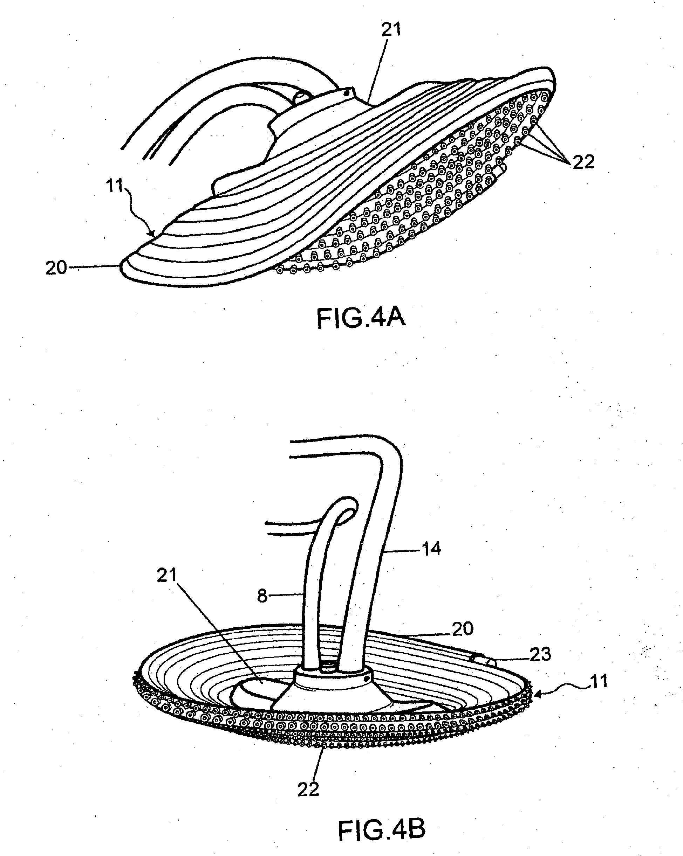

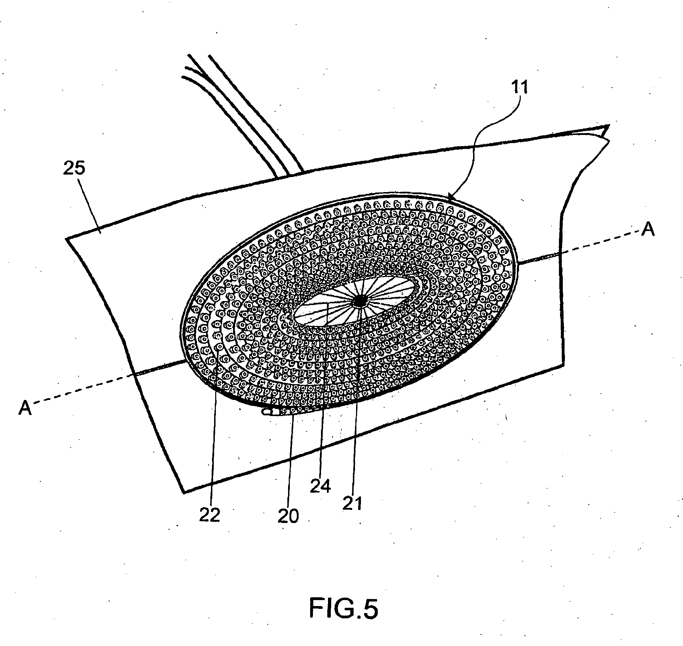

Figures 4 and 5 show in greater detail spiral diffuser 11. Spiral diffuser 11 consists in a coiled tube 20 which can be unrolled and cut according to wound dimensions. The coils of tube 20 are tangentially connected to each other; when, in order to reduce the dimensions of spiral diffuser 11, tube 20 is unrolled, the external coil must be detached from the internal coil. Once the desired dimension of spiral diffuser 11 is determined by cutting the exceeding tube, tube 20 is closed at its distal end with a plug 23. 21 indicates the suction manifold in its entirety, which is internally connected to tube 14. The suction manifold 21 exhibits grooves 24 in order to distribute negative pressure on the wound bed. Tube 20 shows on its inferior surface a very high number of protrusions 22 (e.g., a protrusion every 2 mm). Such protrusions 22 have a tapered shape with an external maximal diameter of about 1,5 mm and a depth of about 1,5 mm. Roughly, at least 9 protrusions should be present for each square centimeter.

The hole in the protrusion under chamber pressure and negative pressure is closed, and opens only under the positive pressure exerted by pump 2 in order to administer medicated liquids. The protrusions moreover increase the contact surface between the spiral diffuser 11 and wound 26.

Figure 5 shows an inferior view of spiral diffuser 11, which can be subdivided into two distinct areas: a first central area 21 and a second area, forming an annulus around the first area, made by tube 20 coils. Grooves 24 in central area 21 distribute negative pressure in a more diffused way on the wound bed. Tube 20, instead, actively instills medications through protrusions 22. Protrusions 22, moreover, further diffuse negative pressure on wound bed and increase the surface in contact with the wound, favoring its healing.

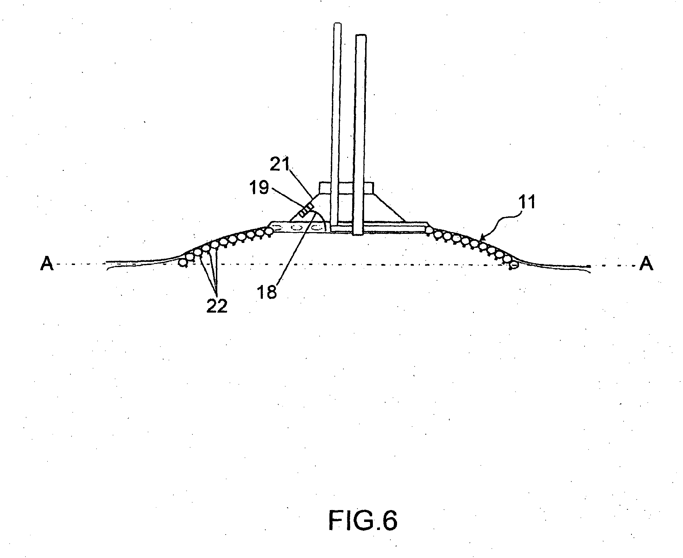

In Figure 6 a vertical section of spiral diffuser is shown according to line A-A shown in Figure 5. The proximity of coils in the spiral diffuser, the partial connection between coils, the free space between coils, all contribute to the diffusion of vacuum on all the wound bed, as shown in Figure 5.

In the section of suction manifold 21 capillary tube 18 is visible, communicating with environment through antibacterial filter 19.

In an alternative embodiment, tube 8 can be connected to a device administering gas under pressure, e.g. oxygen (O₂) or oxygen/ozone mixtures (O₂/O₃). In this case, tube 8 and tube 20 are filled with gas.

## Claims

1. Device for treating wounds with negative pressure, comprising:
- an adhesive film (25) that defines a chamber in relation to the wound;
- a vacuum source (16) which can be adjusted to produce negative pressures in the same chamber;
- a canister (6) for collecting liquids drained from the wound;
- a reservoir (1) containing medicated liquids to be instilled in the wound;
- an unit (3) for setting both the negative pressure and the quantity of administered medication;
- a pressure sensor (17) to detect the negative pressure present in said chamber;
- a diffuser (11) mounted on said film, the diffuser (11) being connected both to vacuum source (16) and to a pump (2) and comprising micro-valves for diffusing medicated liquids; and
- a pump (2) administering medicated liquids exerting a pressure capable of opening the micro-valves;
**characterized in that**
- said spiral diffuser (11) mounted on said film is a spiral diffuser (11) comprising a high number of protrusions (22) on a coiled tube (20), said micro-valves being provided one for each protrusion (22) on said coiled tube (20).

2. Device according to claim 1, wherein the micro-valves in protrusions (22) are closed under negative pressure and room pressure, and open only when in tube (20), through pump (2), a sufficient pressure is exerted.

3. Device according to claim 2, moreover comprising in suction manifold (21) a capillary tube (18), at the end of which an antibacterial micro-filter (19) is present.

4. Device according to claim 2, wherein tube (8) must be connected to a device administering gas or gas mixtures under pressure, with controlled flow.

5. Device according to claim 2, further comprising a sensor (27) detecting the status of the would and especially its pH.

6. Device according to claim 2, further comprising an optical sensor (28) detecting the presence of blood in drained liquids exceeding a given threshold.

7. Device according claim 2, further comprising a vacuum sensor (17) detecting the pressure in the wound.

8. Device according one or more of the claims 5 to 7, wherein each sensor can activate an alarm and stop the device when safety threshold are exceeded.

## Patentansprüche

1. Vorrichtung zur Behandlung von Wunden mit Unterdruck aufweisend:
- einen Klebefilm (25), der eine Kammer bezüglich der Wunde bildet;
- eine Vakuumquelle (16), welche eingestellt werden kann, um Unterdrücke in der gleichen Kammer zu erzeugen;
- einen Behälter (6) zum Sammeln von Flüssigkeiten, welche aus der Wunde geleitet wurden;
- einen Auffangbehälter (1) mit medizinischen Flüssigkeiten, die in die Wunde eingeträufelt werden sollen;
- eine Einheit (3) zur Einstellung sowohl des Unterdruckes als auch der Menge der verabreichten Medikamente;
- einen Drucksensor (17), um den Unterdruck in der Kammer zu detektieren;
- einen Diffusor (11), welcher auf dem Film angebracht ist, wobei der Diffusor (11) sowohl mit der Vakuumquelle (16) als auch mit einer Pumpe (2) verbunden ist und Mikroventile zum Verteilen von medizinischen Flüssigkeiten aufweist; und
- eine Pumpe (2) zum Verabreichen von medizinischen Flüssigkeiten, welche einen Druck aufbauen, der ausreichend ist, um die Mikroventile zu öffnen;
**dadurch gekennzeichnet, dass**
- der Spiraldiffusor (11), der auf dem Film befestigt ist, als Spiraldiffusor (11) mit einer großen Anzahl an Vorsprüngen (22) an einer Rohrwendel (20) ausgebildet ist, wobei für jeden Vorsprung (22) auf der Rohrwendel (20) jeweils ein Mikroventil vorgesehen ist.

2. Vorrichtung nach Anspruch 1, wobei die Mikroventile in den Vorsprüngen (22) bei Unterdruck und Raumdruck geschlossen sind und sich erst öffnen, wenn in dem Rohr (20) durch die Pumpe (2) ein ausreichender Druck aufgebaut wird.

3. Vorrichtung nach Anspruch 2, darüber hinaus in einem Ansaugkanal (21) aufweisend ein Kapillarrohr (18), an dessen Ende sich ein antibakterieller Mikrofilter (19) befindet.

4. Vorrichtung nach Anspruch 2, wobei das Rohr (8) mit einer Vorrichtung zum Verabreichen von Gas oder Gasmischungen unter Druck mit gesteuertem Fluss verbunden sein muss.

5. Vorrichtung nach Anspruch 2, ferner aufweisend einen Sensor (27) zum Erfassen des Status der Wunde und insbesondere ihres pH.

6. Vorrichtung nach Anspruch 2, ferner aufweisend einen optischen Sensor (28) zum Detektieren von Blut in den abgeleiteten Flüssigkeiten, dessen Menge einen gegebenen Schwellwert übersteigt.

7. Vorrichtung nach Anspruch 2, ferner aufweisend einen Unterdrucksensor (17) zum Detektieren des Druckes in der Wunde.

8. Vorrichtung nach einem oder mehreren der Ansprüche 5 bis 7, wobei jeder Sensor geeignet ist, einen Alarm auszulösen und die Vorrichtung anzuhalten, wenn ein Sicherheitsschwellwert überschritten wird.

## Revendications

1. Dispositif pour traiter des plaies par pression négative, comprenant :
- un film adhésif (25) qui définit une chambre reliée à la plaie ;
- une source de vide (16) qui peut être réglée pour produire des pressions négatives dans cette chambre ;
- un récipient (6) destiné à recueillir des liquides drainés provenant de la plaie ;
- un réservoir (1) contenant des liquides médicamenteux destinés à être instillés dans la plaie ;
- une unité (3) pour régler à la fois la pression négative et la quantité de médicament administrée ;
- un capteur de pression (17) destiné à détecter la pression négative présente dans la chambre ;
- un diffuseur (11) monté sur ledit film, le diffuseur (11) étant relié à la fois à la source de vide (16) et à une pompe (2) et comprenant des micro-valves destinées à diffuser les liquides médicamenteux; et
- une pompe (2) d'administration de liquides médicamenteux exerçant une pression capable d'ouvrir les micro-valves ;
**caractérisé en ce que**
- ledit diffuseur en spiral (11) monté sur ledit film est un diffuseur en spiral (11) comprenant un nombre élevé de saillies (22) sur un tube enroulé (20), lesdites micro-valves étant prévues une pour chaque saillie (22) sur ledit tube enroulé (20).

2. Dispositif selon la revendication 1, dans lequel les micro-valves dans les saillies (22) sont fermées sous pression négative et pressions atmosphérique, et ouvertes uniquement lorsque dans le tube (20), à travers la pompe (2), une pression suffisante est exercée.

3. Dispositif selon la revendication 2, comprenant en outre dans le collecteur d'aspiration (21) un tube capillaire (18), à l'extrémité duquel un micro-filtre (19) antibactérien est présent.

4. Dispositif selon la revendication 2, dans lequel le tube (8) doit être relié à un dispositif d'administration de gaz ou mélange de gaz sous pression, avec un débit contrôlé.

5. Dispositif selon la revendication 2, comprenant en outre un capteur (27) destiné à détecter l'état de la plaie et en particulier son pH.

6. Dispositif selon la revendication 2, comprenant en outre un capteur optique (28) destiné à détecter la présence de sang dans les liquides drainés dépassant un seuil donné.

7. Dispositif selon la revendication 2, comprenant en outre un capteur de vide (17) destiné à détecter la pression dans la plaie.

8. Dispositif selon une ou plusieurs des revendications 5 à 7, dans lequel chaque capteur peut activer une alarme et arrêter le dispositif lorsque le seuil de sécurité est dépassé.
